# EUROPEAN PATENT APPLICATION

(11) **EP 4 837 955 A1**
(43) Date of publication of application: **11.11.2026**
(21) Application number: 26175356.0
(22) Date of filing: 29.04.2026
(51) Int. Cl.: C12M 1/42, A61K 35/17, B01L 3/00, B03C 5/02, C12M 1/00

(54) **CONTACTLESS PROCESSING APPARATUS, IMMUNOCYTE ACTIVATION DEVICE, AND ACTIVATION STRUCTURE OF IMMUNOCYTE ACTIVATION DEVICE**

(30) Priority: 05.05.2025 US 202563799764 P
(71) Applicant: Cytoaurora Biotechnologies, Inc., Zhubei City, Hsinchu County (TW)
(72) Inventor: HUANG, Chung-Er, Zhubei City, Hsinchu County (TW); CHEN, Chien-Hung, Zhubei City, Hsinchu County (TW); HUANG, Yen-Sen, Zhubei City, Hsinchu County (TW); HUANG, Yun-Yen, Zhubei City, Hsinchu County (TW)
(74) Representative: Viering, Jentschura & Partner mbB Patent- und Rechtsanwälte

(57) **Abstract**

A contactless processing apparatus (1000), an immunocyte activation device (100), and an activation structure (1) of an immunocyte activation device (100). The activation structure (1) includes a plurality of nanopillars (1411, 1421) each having an activation end (1412, 1422). The activation ends (1412, 1422) of the nanopillars (1411, 1421) define a plurality of first tread surfaces (1414) and a plurality of second tread surfaces (1424), and each of the first tread surfaces (1414) and an adjacent one of the second tread surfaces (1424) have a height difference (S1) along a thickness direction (H). The activation structure (1) can be driven to enable charges to concentrated on the first tread surfaces (1414) and the second tread surfaces (1424), such that at least one immunocyte (P) is attracted onto at least one of the first tread surfaces (1414) and/or at least one of the second tread surfaces (1424) and is activated by contacting the corresponding activation ends (1412, 1422).

## Description

### FIELD OF THE INVENTION

The present invention relates to a cell processing apparatus, and more particularly to a contactless processing apparatus, an immunocyte activation device, and an activation structure of an immunocyte activation device.

### BACKGROUND OF THE INVENTION

A conventional cell processing device, though equipped with a contactless cell sorting mechanism, does not provide an activation structure specifically for immunocytes in the cell sorting mechanism.

### SUMMARY OF THE INVENTION

In response to the above-referenced technical inadequacies, the present invention provides a contactless processing apparatus, an immunocyte activation device, and an activation structure of an immunocyte activation device for effectively improving on the issues associated with conventional cell processing devices.

In order to solve the above-mentioned problems, one of the technical aspects adopted by the present invention is to provide a contactless processing apparatus, which includes an immunocyte activation device and a power device. The immunocyte activation device is configured to accommodate a liquid specimen having a plurality of immunocytes, and the immunocyte activation device includes an activation structure and a mating structure. The activation structure includes a first substrate, a first electrode layer formed on the first substrate, and a nanopillar group. The nanopillar group is arranged on an inner side of the activation structure and has a stepped distribution. The nanopillar group includes a plurality of first sets and a plurality of second sets. The plurality of first sets are staggered with respect to one another and each includes a plurality of first nanopillars. Each of the plurality of first nanopillars has a first activation end. The first activation ends of the plurality of first nanopillars of each of the plurality of first sets jointly define one of a plurality of first tread surfaces. The plurality of second sets are staggered with respect to one another and each includes a plurality of second nanopillars. Each of the plurality of second nanopillars has a second activation end. The second activation ends of the plurality of second nanopillars of each of the second sets jointly define one of a plurality of second tread surfaces. Each of the plurality of second tread surfaces is higher than an adjacent one of the plurality of first tread surfaces by a first height difference along a thickness direction. The first height difference is less than an outer diameter of any one of the plurality of immunocytes. The mating structure is spaced apart from the activation structure along the thickness direction. The mating structure includes a second substrate and a second electrode layer that is formed on the second substrate. The power device is electrically coupled to the first electrode layer and the second electrode layer. The power device is configured to drive the activation structure to concentrate a plurality of charges on the plurality of first tread surfaces and the plurality of second tread surfaces, so that each of the plurality of immunocytes is attracted to move to at least one of the plurality of first tread surfaces and/or at least one of the plurality of second tread surfaces and is activated by contacting the first activation end of at least one of the plurality of first nanopillars and/or the second activation end of at least one of the plurality of second nanopillars.

In order to solve the above-mentioned problems, another one of the technical aspects adopted by the present invention is to provide an immunocyte activation device, which is configured to accommodate a liquid specimen having a plurality of immunocytes. The immunocyte activation device includes an activation structure and a mating structure. The activation structure includes a first substrate, a first electrode layer formed on the first substrate, and a nanopillar group. The nanopillar group is arranged on an inner side of the activation structure and has a stepped distribution. The nanopillar group includes a plurality of first sets and a plurality of second sets. The plurality of first sets are staggered with respect to one another and each includes a plurality of first nanopillars. Each of the plurality of first nanopillars has a first activation end, and the first activation ends of the plurality of first nanopillars of each of the plurality of first sets jointly define one of a plurality of first tread surfaces. The plurality of second sets are staggered with respect to one another and each includes a plurality of second nanopillars. Each of the plurality of second nanopillars has a second activation end, and the second activation ends of the plurality of second nanopillars of each of the plurality of second sets jointly define one of a plurality of second tread surfaces. Each of the plurality of second tread surfaces is higher than an adjacent one of the plurality of first tread surfaces by a first height difference along a thickness direction. The first height difference is less than an outer diameter of any one of the plurality of immunocytes. The mating structure is spaced apart from the activation structure along the thickness direction. The mating structure includes a second substrate and a second electrode layer that is formed on the second substrate. The activation structure is configured to be driven to concentrate a plurality of charges on the plurality of first tread surfaces and the plurality of second tread surfaces, so that each of the plurality of immunocytes is attracted to move to at least one of the plurality of first tread surfaces and/or at least one of the plurality of second tread surfaces and is activated by contacting the first activation end of at least one of the plurality of first nanopillars and/or the second activation end of at least one of the plurality of second nanopillars.

In order to solve the above-mentioned problems, yet another one of the technical aspects adopted by the present invention is to provide an activation structure of an immunocyte activation device. The activation structure includes a first substrate, a first electrode layer formed on the first substrate, and a nanopillar group. The nanopillar group is arranged on an inner side of the activation structure and has a stepped distribution, and the nanopillar group includes a plurality of first sets and a plurality of second sets. The plurality of first sets are staggered with respect to one another and each includes a plurality of first nanopillars. Each of the plurality of first nanopillars has a first activation end, and the first activation ends of the plurality of first nanopillars of each of the plurality of first sets jointly define one of a plurality of first tread surfaces. The plurality of second sets are staggered with respect to one another and each includes a plurality of second nanopillars. Each of the plurality of second nanopillars has a second activation end, and the second activation ends of the plurality of second nanopillars of each of the plurality of second sets jointly define one of a plurality of second tread surfaces. Each of the plurality of second tread surfaces is higher than an adjacent one of the plurality of first tread surfaces by a first height difference along a thickness direction. The first height difference is within a range from 3 µm to 12 µm. The activation structure is configured to be driven to concentrate a plurality of charges on the plurality of first tread surfaces and the plurality of second tread surfaces, so that at least one immunocyte is attracted to move to at least one of the plurality of first tread surfaces and/or at least one of the plurality of second tread surfaces and is activated by contacting the first activation end of at least one of the plurality of first nanopillars and/or the second activation end of at least one of the plurality of second nanopillars.

Therefore, in the contactless processing apparatus, the immunocyte activation device, and the activation structure provided by the present invention, the first height difference between each of the first tread surfaces and the adjacent second tread surface is formed for facilitating the separation of the immunocytes by the first tread surfaces and the second tread surfaces, thereby effectively activating each of the immunocytes through the first activation ends and the second activation ends.

These and other aspects of the present invention will become apparent from the following description of the embodiment taken in conjunction with the following drawings and their captions, although variations and modifications therein can be effected without departing from the scope of the novel concepts of the invention.

### BRIEF DESCRIPTION OF THE DRAWINGS

The described embodiments can be better understood by reference to the following description and the accompanying drawings, in which:
FIG. 1 is a schematic perspective view of a contactless processing apparatus according to a first embodiment of the present invention;
FIG. 2 is a schematic perspective cross-sectional view of FIG. 1;
FIG. 3 is a schematic cross-sectional view taken along line III-III of FIG. 1;
FIG. 4 is a schematic cross-sectional view taken along line IV-IV of FIG. 1;
FIG. 5 is a schematic enlarged view of region V of FIG. 4;
FIG. 6 is a schematic view of a first variation of a nanopillar group shown in FIG. 5;
FIG. 7 is a schematic view of a second variation of the nanopillar group shown in FIG. 5;
FIG. 8 is a schematic view of a third variation of the nanopillar group shown in FIG. 5;
FIG. 9 is a schematic view showing a subsequent operation of the contactless processing apparatus shown in FIG. 3;
FIG. 10 is a schematic view of a subsequent operation of the contactless processing apparatus shown in FIG. 4;
FIG. 11 is a schematic cross-sectional view of the contactless processing apparatus according to a second embodiment of the present invention; and
FIG. 12 is a schematic view of a subsequent operation of the contactless processing apparatus shown in FIG. 11.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

### [First Embodiment]

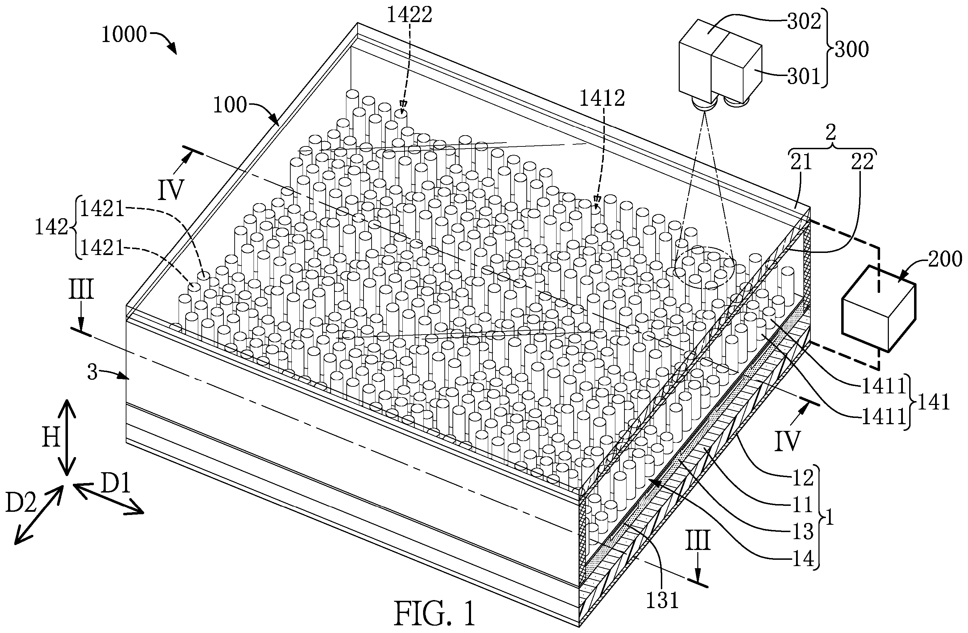

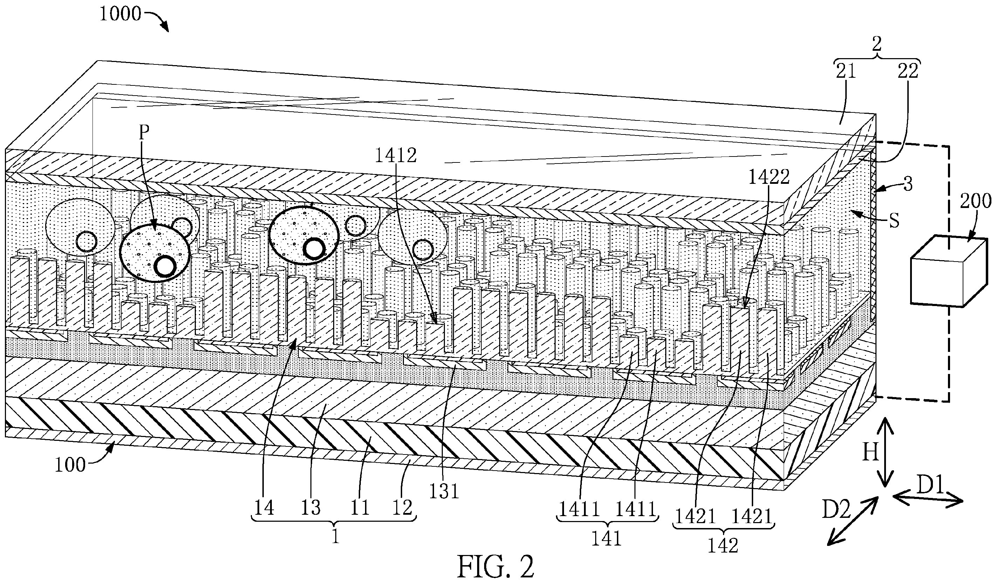

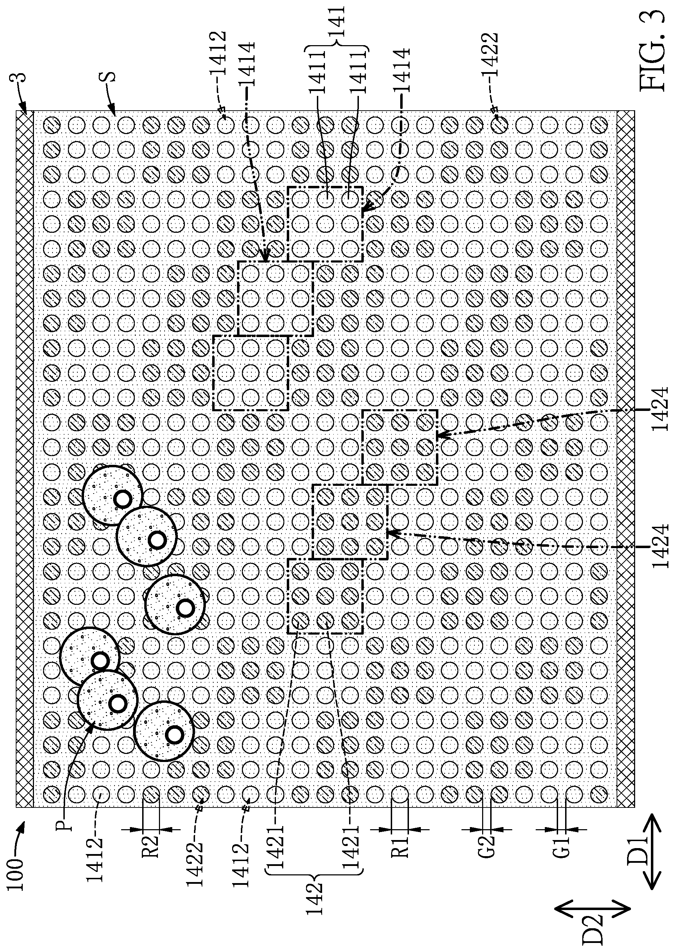

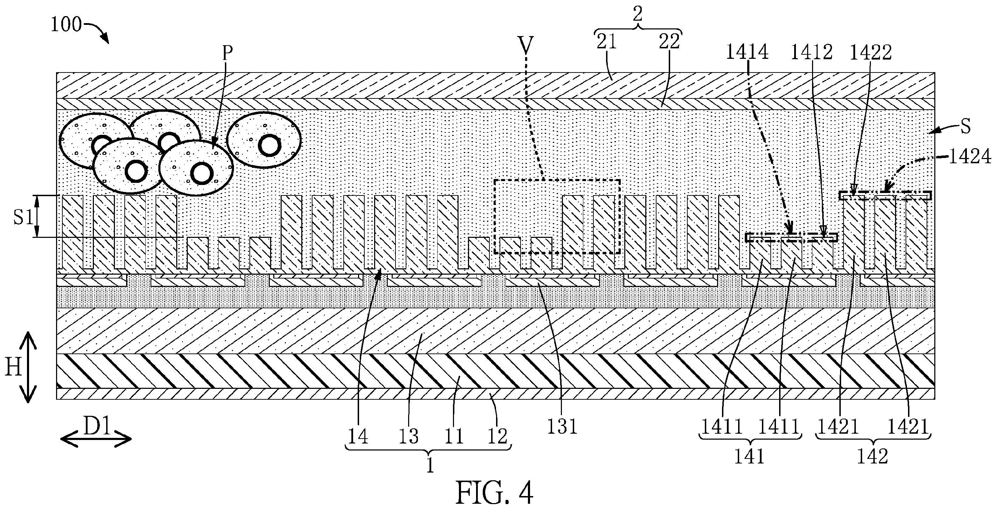

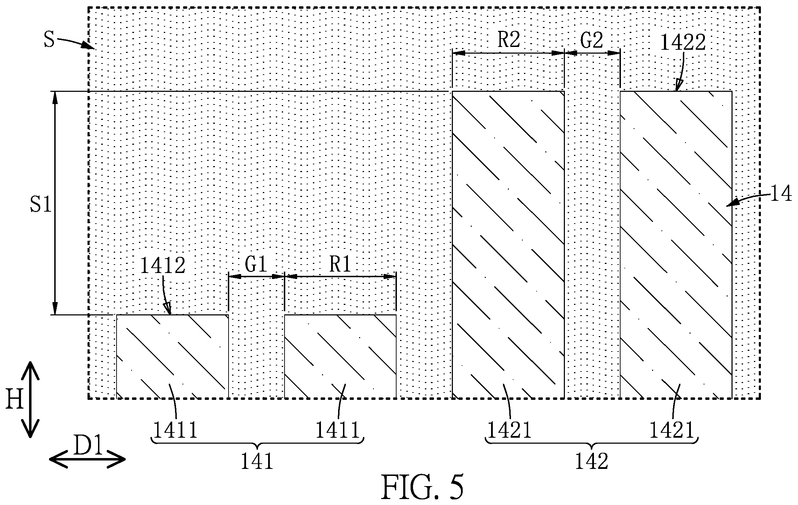

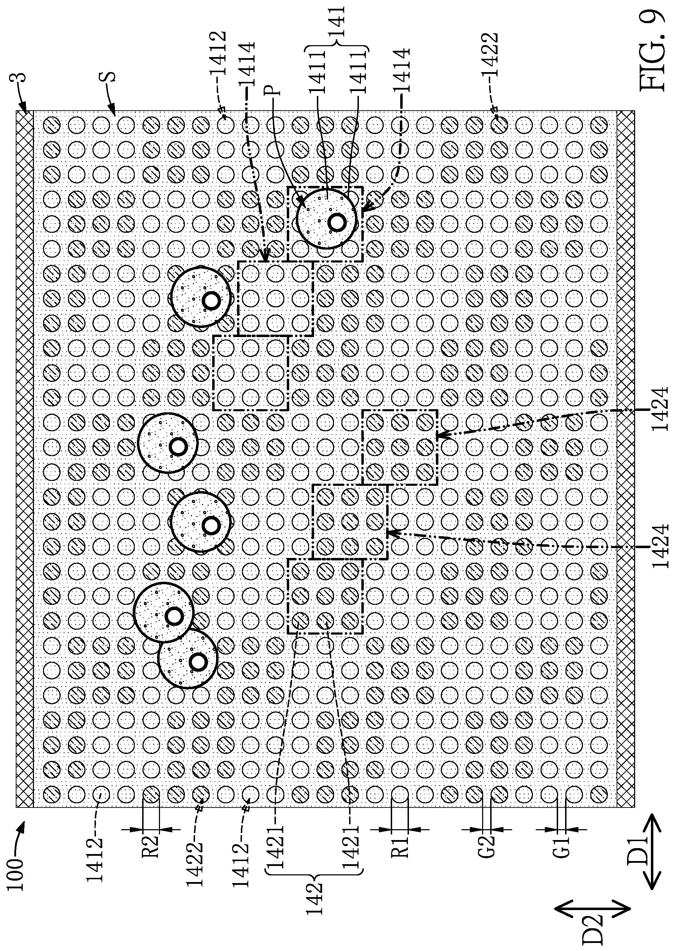

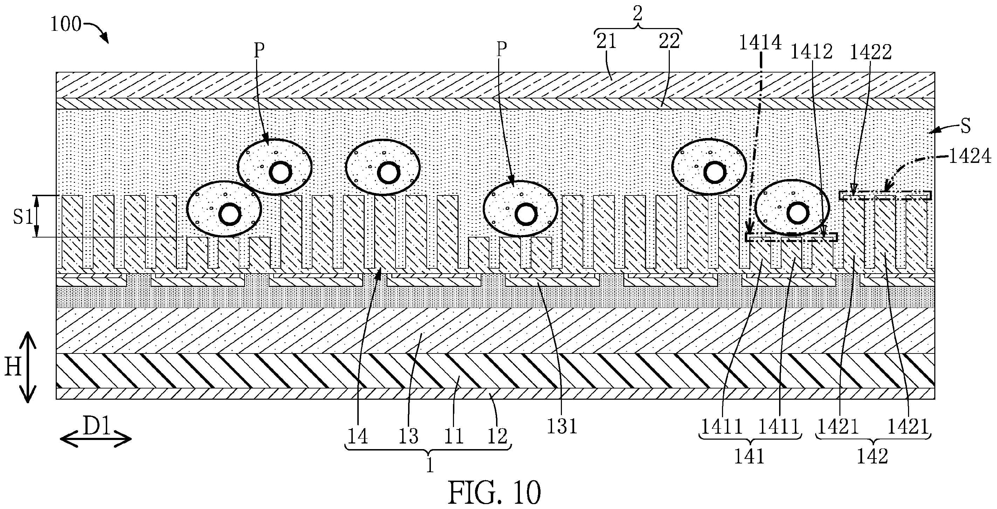

Referring to FIG. 1 to FIG. 10, a first embodiment of the present invention is provided. The present embodiment provides a contactless processing apparatus 1000 including an immunocyte activation device 100, a power device 200 electrically coupled to the immunocyte activation device 100, and a light driving device 300 that corresponds in position to the immunocyte activation device 100, but the present invention is not limited thereto. For example, in other embodiments of the present invention not shown in the drawings, the contactless processing apparatus 1000 can optionally omit the light driving device 300; or, the immunocyte activation device 100 can be independently used (e.g., sold) or can be used in cooperation with other components according to practical requirements.

In the present embodiment, the immunocyte activation device 100 is a chip-scale rectangular structure, and the immunocyte activation device 100 is configured to accommodate a liquid specimen S having a plurality of immunocytes P. In other words, any device not applied to immunocytes is different from the immunocyte activation device 100 provided by the present embodiment.

The immunocyte activation device 100 includes an activation structure 1, a mating structure 2 spaced apart from the activation structure 1 along a thickness direction H, and a bonding layer 3 that is connected to a periphery of the activation structure 1 and a periphery of the mating structure 2. At least one of the activation structure 1 and the mating structure 2 is transparent, and the activation structure 1 and the mating structure 2 in the present embodiment are two plate-like structures that are parallel to each other and that are spaced apart from each other by a distance greater than a dimension of any one of the immunocytes P, but the present invention is not limited thereto.

Specifically, the activation structure 1 in the present embodiment has a thickness within a range from 600 µm to 750 µm, and the activation structure 1 includes a first substrate 11, a first electrode layer 12 formed on the first substrate 11, a photoelectric layer 13 formed on the first substrate 11, and a nanopillar group 14 that is formed on the photoelectric layer 13 (e.g., the photoelectric layer 13 being located between the first substrate 11 and the nanopillar group 14), but the present invention is not limited thereto. For example, in other embodiments of the present invention not shown in the drawings, the photoelectric layer 13 of the activation structure 1 can also be omitted or replaced with another structure according to practical requirements, and the activation structure 1 can be independently used or can be used in cooperation with other components.

In the present embodiment, the first electrode layer 12 is formed on a bottom side of the first substrate 11, the photoelectric layer 13 is formed on a top side of the first substrate 11, and the photoelectric layer 13 has a plurality of transistors 131 arranged in a matrix arrangement. The photoelectric layer 13 can adopt an NPN transistor configuration, a PNP transistor configuration, an NP diode configuration, or a PN diode configuration according to practical requirements, but the present invention is not limited thereto. In other words, according to practical requirements, the photoelectric layer 13 (e.g., the transistors 131) provided by the present embodiment can be driven to provide a corona discharge function for the immunocytes P.

The mating structure 2 includes a second substrate 21 and a second electrode layer 22 that is formed on the second substrate 21and that faces the activation structure 1 (e.g., the photoelectric layer 13 and the nanopillar group 14). In the present embodiment, the power device 200 (e.g., an AC power device) is electrically coupled to the first electrode layer 12 of the activation structure 1 and the second electrode layer 22 of the mating structure 2, so that the light driving device 300 is capable of emitting light toward the activation structure 1 to form, through the photoelectric layer 13, a dielectrophoresis (DEP) pattern (not shown in the drawings) for moving any one of the immunocytes P.

For example, the light driving device 300 can include a camera 301 and a light source 302 that is in cooperation with the camera 301. The light driving device 300 is capable of emitting the light through the light source 302 onto the activation structure 1, so that the activation structure 1 (or the photoelectric layer 13) forms the DEP pattern, whereby any one of the immunocytes P within the liquid specimen S can be moved through the DEP pattern.

To facilitate an understanding of the present embodiment, the above description describes the general cooperative relationship among the immunocyte activation device 100, the power device 200, and the light driving device 300. The following description further describes the nanopillar group 14 of the activation structure 1.

The nanopillar group 14 is arranged on an inner side of the activation structure 1 and has a stepped distribution, and the nanopillar group 14 includes a plurality of first sets 141 staggered with respect to one another and a plurality of second sets 142 that are staggered with respect to one another. The first sets 141 are also staggered with respect to the second sets 142.

Each of the first sets 141 includes a plurality of first nanopillars 1411, a first outer diameter R1 of each of the first sets 141 is substantially within a range from 4 µm to 6 µm, and a first distance G1 between any two of the first nanopillars 1411 adjacent to each other in each of the first sets 141 is within a range from 0.1 µm to 1 µm. Furthermore, the first distance G1 is preferably within a range from 0.2 µm to 0.5 µm, but the present invention is not limited thereto.

In the present embodiment, the first sets 141 are of substantially a same configuration, and the first nanopillars 1411 of each of the first sets 141 are of a same structure and are in a first matrix arrangement, but the present invention is not limited thereto. For example, in other embodiments of the present invention not shown in the drawings, the first sets 141 can adopt configurations different from one another; or, the first nanopillars 1411 of each of the first sets 141 can have slightly different structures or can be in a non-matrix arrangement (e.g., a polygonal arrangement of at least five sides).

Specifically, the first matrix arrangement in the present embodiment has a M number of rows along a first direction D1 perpendicular to the thickness direction H, and a N number of columns along a second direction D2 perpendicular to both the thickness direction H and the first direction D1. Each of M and N is a positive integer greater than 1. In the present embodiment, M equals N and can be within a range from 2 to 10, and both M and N are illustrated as 3 in the drawings, but the present invention is not limited thereto.

In other words, in two of the first sets 141 adjacent to each other along the first direction D1, a portion of the first nanopillars 1411 of one of the two of the first sets 141 and a portion of the first nanopillars 1411 of another one of the two of the first sets 141 are arranged side by side in a L number of columns. Specifically, L is a positive integer less than N (e.g., L in the present embodiment is 1 or 2).

Furthermore, among three of the first sets 141 adjacent to one another along the first direction D1, a portion of the first nanopillars 1411 of a middle one of the three of the first sets 141 and a portion of the first nanopillars 1411 of another one of the three of the first sets 141 on one side thereof are arranged side by side in a L1 number of columns, and a remaining portion of the first nanopillars 1411 of the middle one of the three of the first sets 141 and a portion of the first nanopillars 1411 of another one of the three of the first sets 141 on another side thereof are arranged side by side in a L2 number of columns. Each of L1 and L2 is a positive integer less than N, and a sum of L1 and L2 equals N (e.g., L1 and L2 in the present embodiment are respectively 1 and 2).

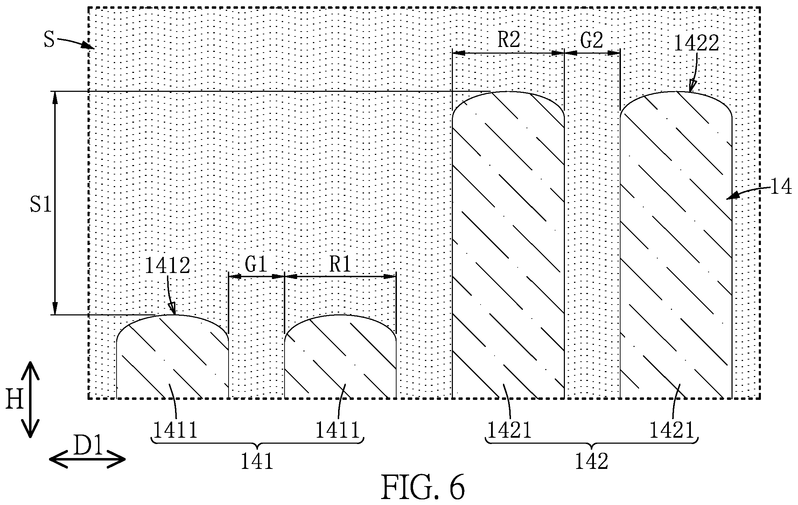

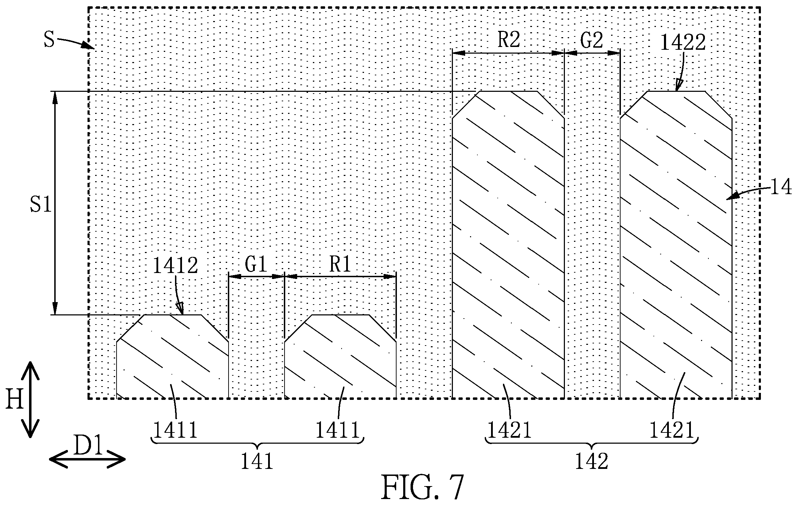

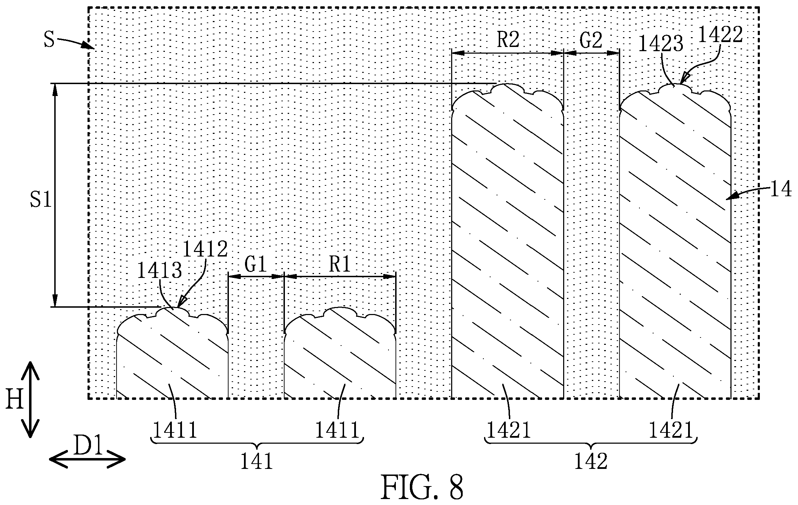

Specifically, each of the first nanopillars 1411 has a first activation end 1412, and the first activation ends 1412 of the first nanopillars 1411 of each of the first sets 141 jointly define a first tread surface 1414. It should be noted that each of the first activation ends 1412 can be formed in a specific shape according to practical requirements (e.g., a conical shape as shown in FIG. 6, a pyramidal shape as shown in FIG. 7, or a plurality of first protrusions 1413 as shown in FIG. 8), and each of the first activation ends 1412 can have an activation substance attached thereto for effectively enhancing the activation effect on the immunocytes P.

Each of the second sets 142 includes a plurality of second nanopillars 1421, and a second outer diameter R2 of each of the second sets 142 is substantially within a range from 4 µm to 6 µm. In the present embodiment, the second outer diameter R2 is substantially equal to the first outer diameter R1. A second distance G2 between any two of the second nanopillars 1421 adjacent to each other in each of the second sets 142 is within a range from 0.1 µm to 1 µm. Moreover, the second distance G2 is preferably within a range from 0.2 µm to 0.5 µm, but the present invention is not limited thereto.

In the present embodiment, the second sets 142 are of substantially a same configuration, and the second nanopillars 1421 of each of the second sets 142 are of a same structure and are arranged in a second matrix arrangement, but the present invention is not limited thereto. For example, in other embodiments of the present invention not shown in the drawings, the second sets 142 can adopt configurations different from one another; or, the second nanopillars 1421 of each of the second sets 142 can have slightly different structures or can be in a non-matrix arrangement (e.g., a polygonal arrangement of at least five sides).

Specifically, the second matrix arrangement in the present embodiment has a M number of rows along the first direction D1 and a N number of columns along the second direction D2. In other words, the second matrix arrangement in the present embodiment is described as being substantially the same as the first matrix arrangement, but the present invention is not limited thereto. For example, in other embodiments of the present invention not shown in the drawings, the second matrix arrangement can be different from the first matrix arrangement according to practical requirements.

In other words, in two of the second sets 142 adjacent to each other along the first direction D1, a portion of the second nanopillars 1421 of one of the two of the second sets 142 and a portion of the second nanopillars 1421 of another one of the two of the second sets 142 are arranged side by side in a L number of columns. Specifically, L is a positive integer less than N (e.g., L in the present embodiment is 1 or 2).

Furthermore, among three of the second sets 142 adjacent to one another along the first direction D1, a portion of the second nanopillars 1421 of a middle one of the three of the second sets 142 and a portion of the second nanopillars 1421 of another one of the three of the second sets 142 on one side thereof are arranged side by side in a L1 number of columns, and a remaining portion of the second nanopillars 1421 of the middle one of the three of the second sets 142 and a portion of the second nanopillars 1421 of another one of the three of the second sets 142 on another side thereof are arranged side by side in a L2 number of columns. Each of L1 and L2 is a positive integer less than N, and a sum of L1 and L2 equals N (e.g., L1 and L2 in the present embodiment are respectively 1 and 2).

Specifically, each of the second nanopillars 1421 has a second activation end 1422, and the second activation ends 1422 of the second nanopillars 1421 of each of the second sets 142 jointly define a second tread surface 1424. It should be noted that each of the second activation ends 1422 can be formed in a specific shape according to practical requirements (e.g., a conical shape as shown in FIG. 6, a pyramidal shape as shown in FIG. 7, or a plurality of second protrusions 1423 as shown in FIG. 8), and each of the second activation ends 1422 can have an activation substance attached thereto for effectively enhancing the activation effect on the immunocytes P.

The first tread surfaces 1414 and the second tread surfaces 1424 are staggered with respect to one another, and an outer contour of any one of the first tread surfaces 1414 and the second tread surfaces 1424 can be slightly larger than the dimension of the immunocytes P, which further facilitates the separation of the immunocytes P, but the present invention is not limited thereto.

Furthermore, each of the first tread surfaces 1414 is higher than the adjacent second tread surface 1424 with respect to the photoelectric layer 13 by a first height difference S1 along the thickness direction H, and the first height difference S1 is less than an outer diameter of any one of the immunocytes P. In other words, the first height difference S1 is within a range from 3 µm to 12 µm, and the first height difference S1 is preferably within a range from 6 µm to 8 µm, but the present invention is not limited thereto.

Based on the above, the power device 200 is configured to drive the activation structure 1 to concentrate a plurality of charges on the first tread surfaces 1414 and the second tread surfaces 1424, so that each of the immunocytes P is attracted to move to at least one of the first tread surfaces 1414 and/or at least one of the second tread surfaces 1424 and is activated by contacting the first activation end 1412 of at least one of the first nanopillars 1411 and/or the second activation end 1422 of at least one of the second nanopillars 1421.

Accordingly, the contactless processing apparatus 1000 in the present embodiment is capable of utilizing the first height difference S1 formed between each of the first tread surfaces 1414 and the adjacent second tread surface 1424 to facilitate the separation of the immunocytes P by the first tread surfaces 1414 and the second tread surfaces 1424, thereby effectively activating each of the immunocytes P through the first activation ends 1412 and the second activation ends 1422.

In addition, the photoelectric layer 13 can be used to concentrate the charges on the first tread surfaces 1414 and the second tread surfaces 1424 by receiving an external ambient light; or, the photoelectric layer 13 can be used to concentrate the charges on the first tread surfaces 1414 and the second tread surfaces 1424 by receiving light emitted from the light driving device 300 onto the photoelectric layer 13, but the present invention is not limited thereto.

### [Second Embodiment]

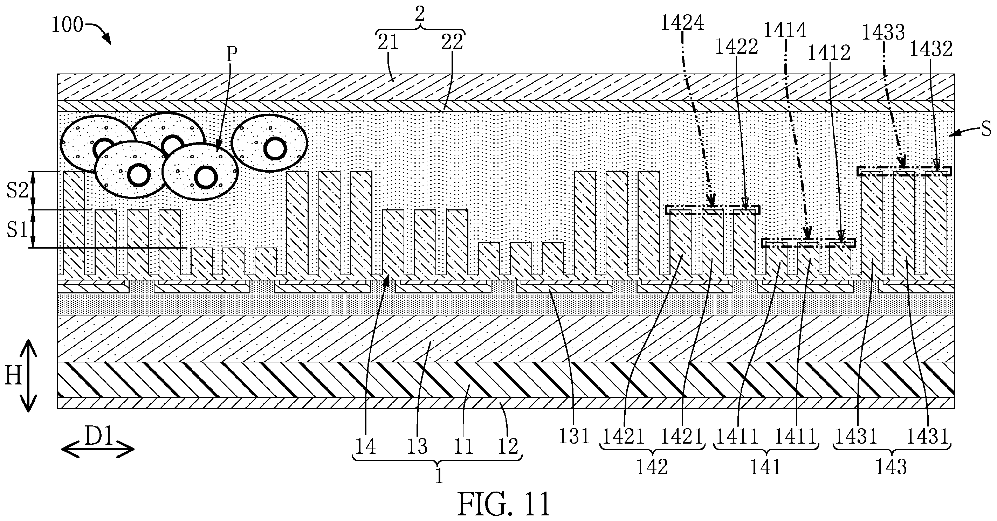

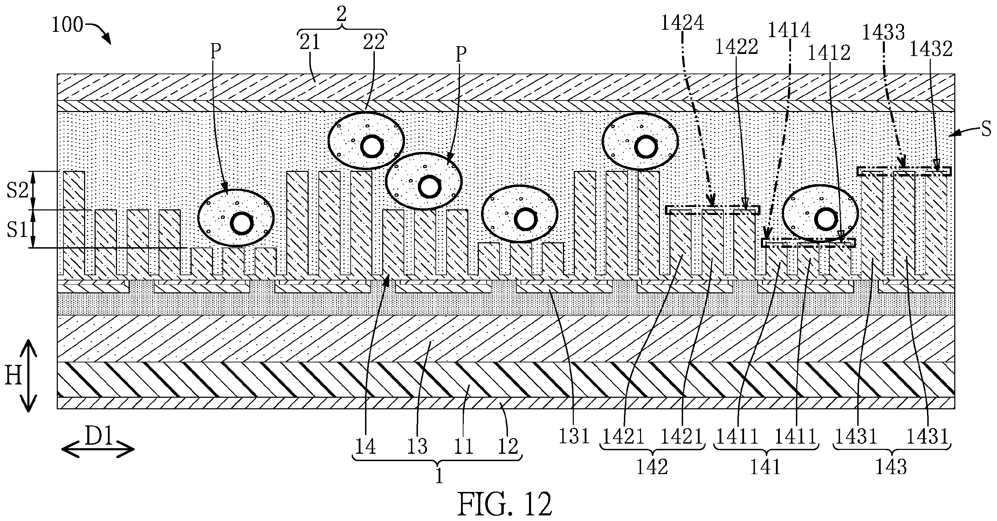

Referring to FIG. 11 and FIG. 12, a second embodiment of the present invention, which is similar to the first embodiment of the present invention, is provided. For the sake of brevity, descriptions of the same components in the first and second embodiments of the present invention will be omitted herein, and the following description only discloses different features between the first and second embodiments.

In the present embodiment, the nanopillar group 14 further includes a plurality of third sets 143, and the third sets 143 are staggered with respect to one another and each includes a plurality of third nanopillars 1431. The third sets 143 are staggered with respect to the first sets 141 and the second sets 142, and the structure of each of the third nanopillars 1431 is similar to that of the first nanopillar 1411 or the second nanopillar 1421, with the only difference being that the height of each of the third nanopillars 1431 is greater than that of the first nanopillar 1411 and also greater than that of the second nanopillar 1421.

Specifically, each of the third nanopillars 1431 has a third activation end 1432, and the third activation ends 1432 of the third nanopillars 1431 of each of the third sets 143 jointly define a third tread surface 1433. Each of the third tread surfaces 1433 is higher than the adjacent second tread surface 1424 with respect to the photoelectric layer 13 by a second height difference S2 along the thickness direction H, the second height difference S2 being within a range from 3 µm to 12 µm (preferably from 6 µm to 8 µm).

### [Beneficial Effects of the Embodiments]

In conclusion, in the contactless processing apparatus, the immunocyte activation device, and the activation structure provided by the present invention, the first height difference between each of the first tread surfaces and the adjacent second tread surface is formed for facilitating the separation of the immunocytes by the first tread surfaces and the second tread surfaces, thereby effectively activating each of the immunocytes through the first activation ends and the second activation ends.

The foregoing description of the exemplary embodiments of the invention has been presented only for the purposes of illustration and description and is not intended to be exhaustive or to limit the invention to the precise forms disclosed. Many modifications and variations are possible in light of the above teaching.

## Claims

1. A contactless processing apparatus (1000), **characterized by** comprising:
an immunocyte activation device (100) configured to accommodate a liquid specimen (S) having a plurality of immunocytes (P), the immunocyte activation device (100) including:
an activation structure (1) including a first substrate (11), a first electrode layer (12) formed on the first substrate (11), and a nanopillar group (14), wherein the nanopillar group (14) is arranged on an inner side of the activation structure (1) and has a stepped distribution, and wherein the nanopillar group (14) includes:
a plurality of first sets (141) staggered with respect to one another and each including a plurality of first nanopillars (1411), wherein each of the plurality of first nanopillars (1411) has a first activation end (1412), and wherein the first activation ends (1412) of the plurality of first nanopillars (1411) of each of the plurality of first sets (141) jointly define one of a plurality of first tread surfaces (1414); and
a plurality of second sets (142) staggered with respect to one another and each including a plurality of second nanopillars (1421), wherein each of the plurality of second nanopillars (1421) has a second activation end (1422), and wherein the second activation ends (1422) of the plurality of second nanopillars (1421) of each of the second sets (142) jointly define one of a plurality of second tread surfaces (1424);
wherein each of the plurality of second tread surfaces (1424) is higher than an adjacent one of the plurality of first tread surfaces (1414) by a first height difference (S1) along a thickness direction (H), and wherein the first height difference (S1) is less than an outer diameter of any one of the plurality of immunocytes (P); and
a mating structure (2) spaced apart from the activation structure (1) along the thickness direction (H), wherein the mating structure (2) includes a second substrate (21) and a second electrode layer (22) that is formed on the second substrate (21); and
a power device (200) electrically coupled to the first electrode layer (12) and the second electrode layer (22), wherein the power device (200) is configured to drive the activation structure (1) to concentrate a plurality of charges on the plurality of first tread surfaces (1414) and the plurality of second tread surfaces (1424), so that each of the plurality of immunocytes (P) is attracted to move to at least one of the plurality of first tread surfaces (1414) and/or at least one of the plurality of second tread surfaces (1424) and is activated by contacting the first activation end (1412) of at least one of the plurality of first nanopillars (1411) and/or the second activation end (1422) of at least one of the plurality of second nanopillars (1421).

2. The contactless processing apparatus (1000) according to claim 1, wherein, in each of the plurality of first sets (141), a first distance (G1) between any two of the plurality of first nanopillars (1411) adjacent to each other is within a range from 0.1 µm to 1 µm, and wherein, in each of the plurality of second sets (142), a second distance (G2) between any two of the plurality of second nanopillars (1421) adjacent to each other is within a range from 0.1 µm to 1 µm.

3. The contactless processing apparatus (1000) according to claim 1, wherein the first activation end (1412) of each of the plurality of first nanopillars (1411) has a plurality of first protrusions (1413), and the second activation end (1422) of each of the plurality of second nanopillars (1421) has a plurality of second protrusions (1423).

4. The contactless processing apparatus (1000) according to claim 1, wherein the plurality of first nanopillars (1411) of each of the plurality of first sets (141) are arranged in a first matrix arrangement having a M number of rows along a first direction (D1) perpendicular to the thickness direction (H) and a N number of columns along a second direction (D2) perpendicular to the thickness direction (H) and the first direction (D1), and wherein each of M and N is a positive integer greater than 1.

5. The contactless processing apparatus (1000) according to claim 4, wherein, in two of the plurality of first sets (141) adjacent to each other along the first direction (D1), a portion of the plurality of first nanopillars (1411) of one of the two of the plurality of first sets (141) and a portion of the plurality of first nanopillars (1411) of another one of the two of the plurality of first sets (141) are arranged side by side in a L number of columns, and wherein L is a positive integer less than N.

6. The contactless processing apparatus (1000) according to claim 4, wherein, among three of the plurality of first sets (141) adjacent to one another along the first direction (D1), a portion of the plurality of first nanopillars (1411) of a middle one of the three of the plurality of first sets (141) and a portion of the plurality of first nanopillars (1411) of another one of the three of the plurality of first sets (141) are arranged side by side in a L1 number of columns, and a remaining portion of the plurality of first nanopillars (1411) of the middle one of the three of the plurality of first sets (141) and a portion of the plurality of first nanopillars (1411) of the other one of the three of the plurality of first sets (141) are arranged side by side in a L2 number of columns, and wherein each of L1 and L2 is a positive integer less than N, and a sum of L1 and L2 equals N.

7. The contactless processing apparatus (1000) according to claim 4, wherein the plurality of second nanopillars (1421) of each of the plurality of second sets (142) are arranged in a second matrix arrangement having a M number of rows along the first direction (D1) and a N number of columns along the second direction (D2).

8. The contactless processing apparatus (1000) according to claim 7, wherein, in two of the plurality of second sets (142) adjacent to each other along the first direction (D1), a portion of the plurality of second nanopillars (1421) of one of the two of the plurality of second sets (142) and a portion of the plurality of second nanopillars (1421) of another one of the two of the plurality of second sets (142) are arranged side by side in a L number of columns, and wherein L is a positive integer less than N.

9. The contactless processing apparatus (1000) according to claim 1, wherein the nanopillar group (14) further includes a plurality of third sets (143) staggered with respect to one another and each including a plurality of third nanopillars (1431), wherein each of the plurality of third nanopillars (1431) has a third activation end (1432), and the third activation ends (1432) of the plurality of third nanopillars (1431) of each of the third sets (143) jointly define one of a plurality of third tread surfaces (1433), and wherein each of the third tread surfaces (1433) is higher than an adjacent one of the second tread surfaces (1424) by a second height difference (S2) along the thickness direction (H), and the second height difference (S2) is within a range from 3 µm to 12 µm.

10. The contactless processing apparatus (1000) according to claim 1, wherein the activation structure (1) has a thickness that is within a range from 600 µm to 750 µm, and the first height difference (S1) is within a range from 3 µm to 12 µm.

11. The contactless processing apparatus (1000) according to claim 1, wherein the activation structure (1) further includes a photoelectric layer (13) located between the first substrate (11) and the nanopillar group (14), and wherein the photoelectric layer (13) is configured to be operated by receiving an external ambient light so as to concentrate the plurality of charges on the plurality of first tread surfaces (1414) and the plurality of second tread surfaces (1424).

12. The contactless processing apparatus (1000) according to claim 1, wherein the activation structure (1) further includes a photoelectric layer (13) located between the first substrate (11) and the nanopillar group (14), and wherein the contactless processing apparatus (1000) further includes:
a light driving device (300) facing the immunocyte activation device (100), wherein the light driving device (300) is configured to emit light toward the activation structure (1) so as to form, through the photoelectric layer (13), a dielectrophoresis (DEP) pattern for moving any one of the immunocytes (P).

13. The contactless processing apparatus (1000) according to claim 11, wherein the activation structure (1) further includes a photoelectric layer (13) located between the first substrate (11) and the nanopillar group (14), and wherein the photoelectric layer (13) is configured to be operated by receiving light so as to concentrate the plurality of charges on the plurality of first tread surfaces (1414) and the plurality of second tread surfaces (1424).

14. An immunocyte activation device (100) configured to accommodate a liquid specimen (S) having a plurality of immunocytes (P), **characterized by** comprising:
an activation structure (1) including a first substrate (11), a first electrode layer (12) formed on the first substrate (11), and a nanopillar group (14), wherein the nanopillar group (14) is arranged on an inner side of the activation structure (1) and has a stepped distribution, and wherein the nanopillar group (14) includes:
a plurality of first sets (141) staggered with respect to one another and each including a plurality of first nanopillars (1411), wherein each of the plurality of first nanopillars (1411) has a first activation end (1412), and the first activation ends (1412) of the plurality of first nanopillars (1411) of each of the plurality of first sets (141) jointly define one of a plurality of first tread surfaces (1414); and
a plurality of second sets (142) staggered with respect to one another and each including a plurality of second nanopillars (1421), wherein each of the plurality of second nanopillars (1421) has a second activation end (1422), and the second activation ends (1422) of the plurality of second nanopillars (1421) of each of the plurality of second sets (142) jointly define one of a plurality of second tread surfaces (1424);
wherein each of the plurality of second tread surfaces (1424) is higher than an adjacent one of the plurality of first tread surfaces (1414) by a first height difference (S1) along a thickness direction (H), and wherein the first height difference (S1) is less than an outer diameter of any one of the plurality of immunocytes (P); and
a mating structure (2) spaced apart from the activation structure (1) along the thickness direction (H), wherein the mating structure (2) includes a second substrate (21) and a second electrode layer (22) that is formed on the second substrate (21);
wherein the activation structure (1) is configured to be driven to concentrate a plurality of charges on the plurality of first tread surfaces (1414) and the plurality of second tread surfaces (1424), so that each of the plurality of immunocytes (P) is attracted to move to at least one of the plurality of first tread surfaces (1414) and/or at least one of the plurality of second tread surfaces (1424) and is activated by contacting the first activation end (1412) of at least one of the plurality of first nanopillars (1411) and/or the second activation end (1422) of at least one of the plurality of second nanopillars (1421).

15. An activation structure (1) of an immunocyte activation device (100), **characterized by** comprising: a first substrate (11), a first electrode layer (12) formed on the first substrate (11), and a nanopillar group (14), wherein the nanopillar group (14) is arranged on an inner side of the activation structure (1) and has a stepped distribution, and the nanopillar group (14) includes:
a plurality of first sets (141) staggered with respect to one another and each including a plurality of first nanopillars (1411), wherein each of the plurality of first nanopillars (1411) has a first activation end (1412), and the first activation ends (1412) of the plurality of first nanopillars (1411) of each of the plurality of first sets (141) jointly define one of a plurality of first tread surfaces (1414); and
a plurality of second sets (142) staggered with respect to one another and each including a plurality of second nanopillars (1421), wherein each of the plurality of second nanopillars (1421) has a second activation end (1422), and the second activation ends (1422) of the plurality of second nanopillars (1421) of each of the plurality of second sets (142) jointly define one of a plurality of second tread surfaces (1424);
wherein each of the plurality of second tread surfaces (1424) is higher than an adjacent one of the plurality of first tread surfaces (1414) by a first height difference (S1) along a thickness direction (H), and wherein the first height difference (S1) is within a range from 3 µm to 12 µm;
wherein the activation structure (1) is configured to be driven to concentrate a plurality of charges on the plurality of first tread surfaces (1414) and the plurality of second tread surfaces (1424), so that at least one immunocyte (P) is attracted to move to at least one of the plurality of first tread surfaces (1414) and/or at least one of the plurality of second tread surfaces (1424) and is activated by contacting the first activation end (1412) of at least one of the plurality of first nanopillars (1411) and/or the second activation end (1422) of at least one of the plurality of second nanopillars (1421).
